Europäisches Patentamt
European Patent Office
Office européen des brevets

(19) (11) Veröffentlichungsnummer: **0 262 552**
**A1**

(12)
# EUROPÄISCHE PATENTANMELDUNG

(21) Anmeldenummer: **87113801.2**

(22) Anmeldetag: **22.09.87**

(51) Int. Cl.⁴: **A61K 37/64**,
//(A61K37/64,31:54)

(30) Priorität: **30.09.86 DE 3633114**

(43) Veröffentlichungstag der Anmeldung:
**06.04.88 Patentblatt 88/14**

(84) Benannte Vertragsstaaten:
**AT BE CH DE ES FR GB GR IT LI LU NL SE**

(71) Anmelder: **HOECHST AKTIENGESELLSCHAFT**
**Postfach 80 03 20**
**D-6230 Frankfurt am Main 80(DE)**

(72) Erfinder: **Becker, Reinhard, Dr.**
**Adelheidstrasse 101**
**D-6200 Wiesbaden(DE)**
Erfinder: **Geiger, Rolf, Prof. Dr.**
**Heinrich-Bleicher-Strasse 33**
**D-6000 Frankfurt am Main(DE)**
Erfinder: **Henning, Rainer, Dr.**
**Rotenhofstrasse 31**
**D-6234 Hattersheim am Main(DE)**
Erfinder: **Teetz, Volker, Dr.**
**An der Tann 20**
**D-6238 Hofheim am Taunus(DE)**
Erfinder: **Urbach, Hansjörg, Dr.**
**Le Lavandoustrasse 41**
**D-6242 Kronberg/Taunus(DE)**

(54) **Pharmazeutische Zubereitung zur Behandlung des Bluthochdrucks.**

(57) Die Erfindung betrifft eine pharmazeutische Zubereitung aus einem Angiotensin-Converting-Enzyme-Inhibitor und einem Diuretikum vom Thiazid-Typ, ein aus diesen Komponenten bestehendes Erzeugnis sowie deren Verwendung bei der Behandlung des Bluthochdrucks.

EP 0 262 552 A1

## Pharmazeutische Zubereitung zur Behandlung des Bluthochdrucks

Bekanntermaßen kann mit Hemmstoffen des Angiotensin-Converting-Enzyms (ACE-Inhibitoren), wie Captopril oder Enalapril, der Bluthochdruck bei essentiellen Hypertonikern gesenkt werden (Therapiewoche 29 [1979] 7746; Lancet 2 [1981] 543-546). Ein bestimmer Prozentsatz essentieller Hypertoniker spricht jedoch auf solche Stoffe nicht an (Drug Devel. Eval. 4 [1980] 82-91).

Es ist bekannt, daß die antihypertensive Wirkung von Enalapril oder Captopril durch Zusatz diuretisch wirksamer Mengen eines Diuretikums vom Thiazid-Typ oder analogen Verbindungen verstärkt wird (Brunner et al., Clin. Exp. Hypertension 2 [1980] 639-657; McGregor et al., Br. Med. J. 284 [1982] 639-696). Als Grund für diesen Effekt wird allgemein angenommen, daß das Diuretikum über einen Salz-und Volumenverlust das Renin-Angiotensin-System stimuliert (P. J. S. Chin et al., J. Pharm. Pharmacol. 37 [1985] 105).

Die Erfindung betrifft pharmazeutische Zubereitungen, enthaltend

a) einen Angiotensin-Converting-Enzyme-Inhibitor der Formel I

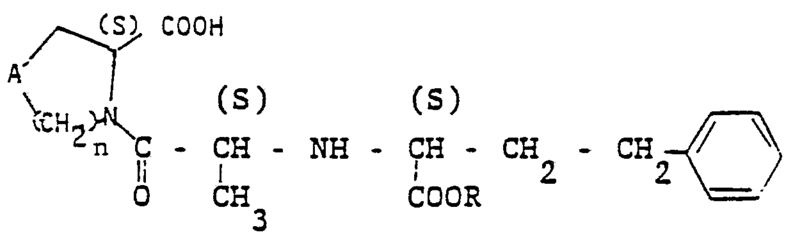

(I)

in welcher
A Cyclopentan-1,2-diyl, Cyclohexan-1,2-diyl oder o-Phenylen und
R Wasserstoff, Methyl, Ethyl oder Benzyl bedeuten und
n 0 oder 1 ist,
oder
dessen physiologisch verträgliches Salz und

b) ein Thiazid der Formel II

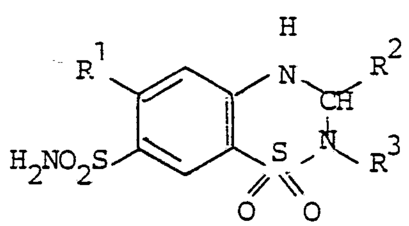

(II)

in welcher
$R^1$ Chlor oder Trifluormethyl,
$R^2$ Wasserstoff, Cyclopentylmethyl, Benzyl, Benzylthiomethyl, Isobutyl, Chlormethyl, Dichlormethyl oder 2,2,2-Trifluorethylthiomethyl und
$R^3$ Wasserstoff oder Methyl bedeuten,
sowie deren physiologisch verträglichen Salze.

Falls A für o-Phenylen steht, ist n vorzugsweise 1, andernfalls ist n vorzugsweise 0.

Bevorzugt sind solche Zubereitungen, die einen ACE-Inhibitor der Formel III, IV oder V,

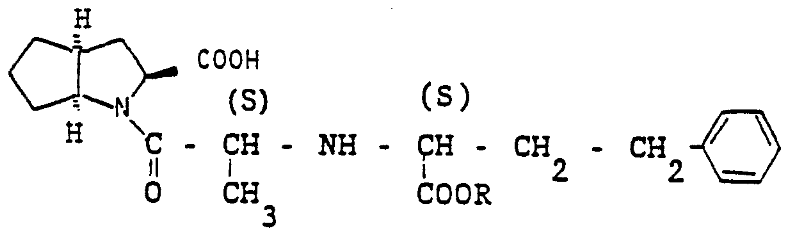 (III)

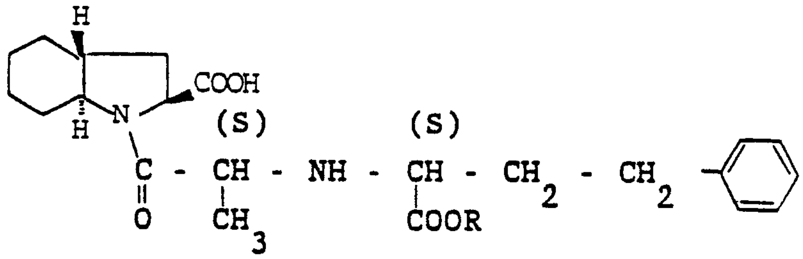 (IV)

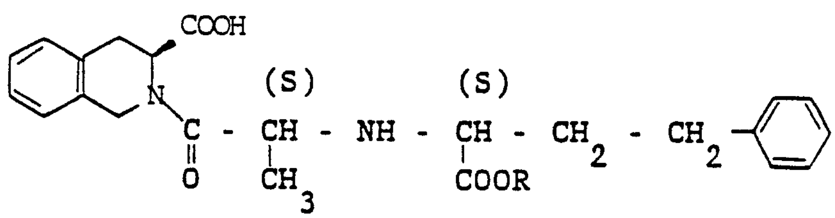 (V)

in welchen
R jeweils Wasserstoff, Methyl, Ethyl oder Benzyl, vorzugsweise Ethyl bedeutet,
enthalten.

ACE-Inhibitoren der Formel I lassen sich herstellen, indem man ihre Fragmente in einem geeigneten Lösungsmittel gegebenenfalls in Gegenwart einer Base und/oder eines Kupplungshilfsmittels miteinander umsetz, gegebenenfalls intermediär entstehende ungesättigte Verbindungen, wie Schiff'sche Basen reduziert und zum Schutz der Carboxygruppe temporär eingefügte Schutzgruppen abspaltet und die erhaltenen Verbindungen gegebenenfalls in ihre physiologisch verträglichen Salze überführt.

In der genannten Weise kann man Verbindungen der Formel VI mit Verbindungen der Formel VII umsetzen.

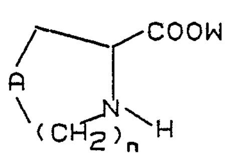

(VI)

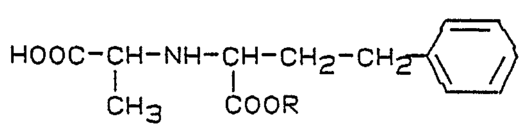

(VII)

Die Umsetzung dieser Verbindungen kann beispielsweise in Analogie zu bekannten Peptidkupplungsverfahren in Gegenwart von Kupplungshilfsmitteln, wie Carbodiimiden (z.B. Dicyclohexylcarbodiimid), Diphenylphosphorylazid, Alkanphosphorsäureanhydriden, Dialkylphosphinsäureanhydriden oder N,N-Succinimidylcarbonate in $CH_3CN$ durchgeführt werden. Aminogruppen in Verbindungen der Formel VI können mit

Tetraethyldiphosphit aktiviert werden. Die Verbindungen der Formel VII können in Aktivester (z.B. mit 1-Hydroxybenzotriazol), gemischte Anhydride, (z.B. mit Chlorameisensäureestern), Azide oder Carbodiimid-Derivate überführt und damit aktiviert werden (vgl. Schröder, Lübke, The Peptides, Band 1, New York, 1965,Seiten 76-136).

Ebenso lassen sich auch Verbindungen der Formel VII' mit Verbindungen der Formel VIII unter Bildung von Verbindungen der Formel I umsetzen,

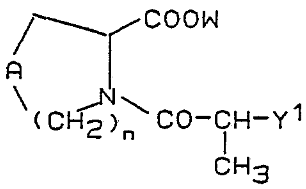

(VII')

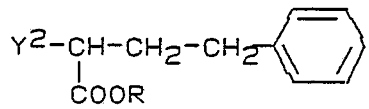

(VIII)

worin entweder $Y^1$ für Amino und $Y^2$ für eine Abgangsgruppe oder $Y^1$ für eine Abgangsgruppe und $Y^2$ für Amino stehen. Geeignete Abgangsgruppen sind z.B. Cl, Br, J, Alkylsulfonyloxy oder Arylsulfonyloxy.

Alkylierungen dieser Art führt man zweckmäßigerweise in Wasser oder einem organischen Lösungsmittel in Gegenwart einer Base durch.

Des weiteren lassen sich Verbindungen der Formel IX mit Verbindungen der Formel X kondensieren.

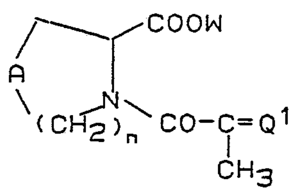

(IX)

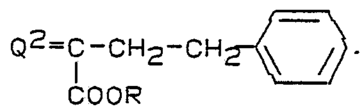

(X)

worin entweder $Q^1$ für Amino + Wasserstoff und $Q^2$ für Oxo steht oder $Q^1$ für Oxo und $Q^2$ für Amino + Wasserstoff steht. Die Kondensation wird zweckmäßigerweise in Wasser oder einem organischen Lösungsmittel, wie einem niederen Alkohol, in Gegenwart eines Reduktionsmittels, wie $NaBH_3CN$, durchgeführt, wobei Verbindungen der Formel I direkt erhalten werden. Man kann aber auch die als Zwischenprodukte entstehenden Schiff'schen Basen oder Enamine gegebenenfalls nach vorheriger Isolierung unter Bildung von Verbindungen der Formel I reduzieren, beispielsweise durch Hydryrierung in Gegenwart eines Übergangsmetallkatalysators.

Schließlich führt auch die Umsetzung von Verbindungen der Formel IX ($Q^1$ = H + $NH_2$) mit Verbindungen der Formel XI oder deren Umsetzung mit Verbindungen der Formeln XII und XIII zu Verbindungen der Formel I,

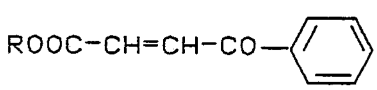

(XI)

OCH- COOR

(XII)

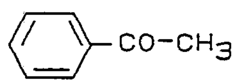

(XIII)

wobei intermediär entstehende Schiff'sche Basen reduziert und eine Carbonylgruppe reduktiv in Methylen überführt werden.

Man führt die oben genannten Umsetzungen in der Regel bei -20°C bis zur Siedetemperatur des Reaktonsgemisches durch.

In den oben genannten Formeln VI - XIII sind A, R und n wie in Formel I definiert. W steht für eine hydrolytisch oder hydrogenolytisch abspaltbare Gruppe, wie ($C_1$-$C_6$)-Alkyl oder Benzyl, die nach beendeter Umsetzung durch Behandeln mit Säure oder Base oder durch Hydrieren abgespalten wird.

Bevorzugte Thiazide sind Hydrochlorothiazid (Formel II; $R^1$ = Chlor, $R^2$ = $R^3$ = Wasserstoff), Trichlormethiazid (Formel II; $R^1$ = Chlor, $R^2$ = Dichlormethyl, $R^3$ = Wasserstoff), Butizid (Formel II; $R^1$ = Chlor, $R^2$ = Isobutyl, $R^3$ = Wasserstoff) und Bendroflumethiazid (Formel II; $R^1$ = Trifluormethyl, $R^2$ = Benzyl, $R^3$ = Wasserstoff), insbesondere Hydrochlorothiazid.

Besonders bevorzugt sind solche Zubereitungen die einen ACE-Inhibitor der Formel III, worin R Ethyl (Ramipril) oder Wasserstoff (Ramiprilat) bedeutet und Hydrochlorothiazid enthalten.

Die Kombination von ACE-Inhibitoren und Thiazid-Diuretikum wirkt stark und anhaltend blutdrucksenkend und kann daher zur Behandlung des Bluthochdruckes verschiedener Genese eingesetzt werden. Von besonderem Interesse ist dabei die Tatsache, daß die Wirkungen der beiden Komponenten sich nicht additiv verhalten; es wird vielmehr ein synergistischer Effekt beobachtet.

Aus den genannten Gründen ist die erfindungsgemäße Zubereitung den Einzelkomponenten in der Behandlung des Bluthochdrucks überlegen, da sie es erlaubt, geringere Dosen der Einzelkomponenten zuzuführen und damit eventuelle toxikologische Probleme zu verringern.

Die Erfindung betrifft auch ein Verfahren zur Herstellung einer solchen Zubereitung, das dadurch gekennzeichnet ist, daß man

a) einen Angiotensin-Converting-Enzyme-Inhibitor oder dessen Salz und

b) ein Thiazid-Diuretikum oder dessen Salz zusammen mit physiologisch annehmbaren Trägern und gegebenenfalls weiteren Hilfs-oder Zusatzstoffen in eine geeignete Darreichungsform bringt.

Die Erfindung betrifft weiterhin ganz allgemein Erzeugnisse, enthaltend

a) einen Angiotensin-Converting-Enzyme-Inhibitor oder dessen physiologisch verträgliches Salz und

b) ein Thiazid-Diuretikum oder dessen physiologisch verträgliches Salz, als Kombinationspräparat zur gleichzeitigen, getrennten oder zeitlich abgestuften Anwendung bei der Behandlung des Bluthochdrucks.

Die Dosen des ACE-Inhibitors und des Thiazid-Diuretikums in den erfindungsgemäßen Zubereitungen bzw. Erzeugnissen werden vorzugsweise jeweils so gewählt, daß der ACE-Inhibitor und/oder das Thiazid-Diuretikum für sich allein noch keine volle Wirkung zeigen würde(n). So genügt bei den Thiazid-Diuretika - schon eine Dosis, die unterhalb des $ED_{50}$-Werts liegt. ACE-Inhibitoren als Komponenten können bereits in Dosen eingesetzt werden, die etwa bei der minimalen, für eine Plasma-ACE-Hemmung ausreichenden Dosis liegen (Bestimmung siehe: Metzger et al., Arzneim.-Forsch./Drug. Res. 34 (II), 1402, 1403); sie können damit unterhalb solcher liegen, die für eine akut blutdrucksenkende Wirkung bei Anwendung eines ACE-Hemmers allein erforderlich sind.

Bei der erfindungsgemäßen Anwendung bei Säugern, vorzugsweise beim Menschen, bewegen sich beispielsweise die Dosen eines ACE-Inhibitors der o. g. Formel I im Bereich von 0,05 bis 5 mg/kg/Tag und die eines Thiazid-Diuretikums der o. g. Formel IV im Bereich von 2,5 bis 50 mg/kg/Tag.

Die erfindungsgemäßen Zubereitungen bzw. Erzeugnisse können parenteral oder oral verabreicht werden. Bevorzugt ist die orale Applikationsform.

Die pharmakologisch verwendbaren Kombinationen der vorliegenden Erfindung und ihre Salze können zur Herstellung von pharmazeutischen Präparaten verwendet werden, welche eine wirksame Menge der Aktivsubstanzen zusammen mit Trägerstoffen enthalten und die sich zur enteralen und parenteralen Verabreichung eignen. Vorzugsweise verwendet werden Tabletten oder Gelatinekapseln, welche die Wirkstoffe zusammen mit Verdünnungsmitteln, z.B. Lactose, Dectrose, Rohrzucker, Mannitol, Sorbitol, Cellulose und/oder Glycin und Gleitmitteln wie Kieselerde, Talk, Stearinsäure oder deren Salze, wie Magnesium-oder Calciumstearat, und/oder Polyethylenglykol enthalten. Tabletten enthalten ebenfalls Bindemittel wie Magnesiumaluminiumsilicat, Stärke, Gelatine Traganth, Methylcellulose, Natriumcarboxymethylcellulose und/oder Polyvinylpyrrolidon und, falls benötigt, Farbstoff, Geschmacksstoffe und Süßmittel. Injizierbare Lösungen sind vorzugsweise isotonische wäßrige Lösungen oder Suspension, die sterilisiert sein können und Hilfsstoffe wie Konservier-, Stabilisierungs-, Netz-und/oder Emulgiermittel, Löslichkeitsvermitteler, Salze zur Regulierung des osmotischen Drucks und/oder Puffersubstanzen enthalten können. Die erfindungsgemäßen pharmazeutischen Präparate, die, wenn erwünscht, weitere pharmakologisch wertvolle Stoffe enthalten können, werden z.B. mittels konventioneller Misch-, Granulier-und Dragierverfahren, hergestellt und enthalten 0,1 % bis etwa 75 %, bevorzugt etwa 1 % bis etwa 50 % der Wirkstoffe.

Die nachstehenden Beispiele sollen die vorliegende Erfindung erläutern, ohne die Erfindung auf die hier stellvertretend genannten Zubereitungen zu beschränken:

Beispiel 1

Herstellung eines oralen Kombinationspräparats aus Ramipril (=A) und Hydrochlorothiazid (=B)

1000 Tabletten, die 1 mg A und 25 mg B enthalten, werden mit den folgenden Hilfsmiteln hergestellt:

A 1 g
B 25 g
Maisstärke 140 g
Gelatine 7,5 g
Mikrokristalline Cellulose 2,5 g
Magnesiumstearat 2,5 g

A und B werden mit einer wäßrigen Gelatine-Lösung gemischt. Die Mischung wird getrocknet und zu einem Granulat vermahlen. Mikrokristalline Cellulose und Magnesiumstearat sowie Maisstärke werden mit dem Granulat vemischt. Das entstehende Granulat wird zu 1000 Tabletten gepreßt, wobei jede Tablette 1 mg A und 25 mg B enthält.

Beispiel 2

Herstellung eines parenteralen Kombinationspräparats aus Ramiprilat (=C; Formel III worin R = Wasserstoff bedeutet) und Trichlormethiazid (=D)

Die Herstellung einer Injektionslösung zur Behandlung der Hypertonie wird im folgenden beschrieben:

C 0,25 g
D 3 g
Methylparaben 5 g
Propylparaben 1 g
Natriumchlorid 25 g
Wasser für Injektion 5 l

A, B, die Konservierungsstoffe und Natriumchlorid werden in Wasser für Injektion gelöst und mit Wasser für Injektion auf 5 l aufgefüllt. Die Lösung wird steril gefiltert und aseptisch in vorsterilisierte Flaschen gefüllt, die mit sterilisierten Gummikappen verschlossen werden. Jede Flasche enthält 5 ml Lösung.

Beispiel 3

Herstellung eines oralen Kombinationspräparats aus Ramipril (=A) und Butizid (=E)

1000 Tabletten, die 3 mg A und 4 mg E enthalten, werden mit den folgenden Hilfsmitteln hergestellt:

A 3 g
E 4 g
Maisstärke 140 g
Gelatine 7,5 g
Mikrokristalline Cellulose 2,5 g
Magnesiumstearat 2,5 g

A und E werden mit einer wäßrigen Gelatine-Lösung gemischt. Die Mischung wird getrocknet und zu einem Granulat vermahlen. Mikrokristalline Cellulose und Magnesiumstearat sowie Maisstärke werden mit den Granulat vermischt. Das entstehende Granulat wird zu 1000 Tabletten gepreßt, wobei jede Tablette 3 mg A und 4 mg E enthält.

Beispiel 4

Herstellung eines oralen Kombinationspräparats aus Ramipril (=A) und Bendroflumethiazid (=F)

1000 Tabletten, die je 2 mg A und 3 mg F enthalten, werden mit den folgenden Hilfsmitteln hergestellt:

A 2 g
F 3 g
Maisstärke 140 g
Gelatine 7,5 g
Mikrokristalline Cellulose 2,5 g
Magnesiumstearat 2,5 g

A und F werden mit einer wäßrigen Gelatine-Lösung gemischt. Die Mischung wird getrocknet und zu einem Granulat vermahlen. Mikrokristalline Cellulose und Magnesiumstearat sowie Maisstärke werden mit dem Granulat vermischt. Das entstehende Granulat wird zu 1000 Tabletten gepreßt, wobei jede Tablette 2mg A und 3 mg F enthält.

Beispiel 5

Herstellung eines oralen Kombinationspräparates aus Ramipril (=A) und Hydrochlorothiazid (=B)

Kapseln, die für die orale Verabreichung geeignet sind, enthalten die nachfolgend genannten Bestandteile und können auf an sich bekannte Weise hergestellt werden, indem man Wirk-und Hilfsstoffe vermischt und in Gelatinekapseln abfüllt.

A 1 mg
B 25 mg
Milchzucker 100 mg
Maisstärke 55 mg
Talkum 3 mg
kolloidales Silizimdioxid 3 mg
Magnesiumstearat 2 mg

**Ansprüche**

1. Pharmazeutische Zubereitung, enthaltend
a) einen Angiotensin-Converting-Enzyme-Inhibitor der Formel I

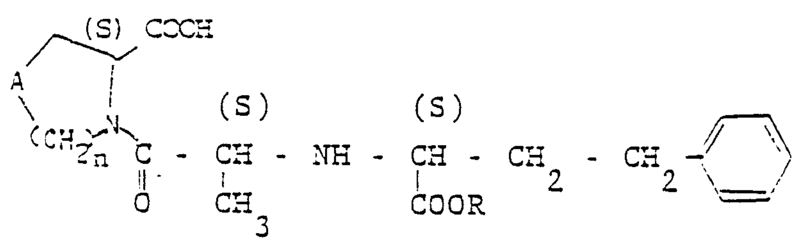

(I)

in welcher
A Cyclopentan-1,2-diyl, Cyclohexan-1,2-diyl oder o-Phenylen und
R Wasserstoff, Methyl, Ethyl oder Benzyl bedeuten und
n 0 oder 1 ist,
oder
dessen physiologisch verträgliches Salz und
b) ein Thiazid der Formel II

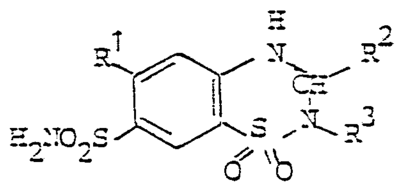

(II)

in welcher
$R^1$ Chlor oder Trifluormethyl,
$R^2$ Wasserstoff, Cyclopentylmethyl, Benzyl, Benzylthiomethyl, Isobutyl, Chlormethyl, Dichlormethyl oder 2,2,2-Trifluorethylthiomethyl und
$R^3$ Wasserstoff oder Methyl bedeuten,
oder dessen physiologisch verträgliches Salz.

2. Zubereitung gemäß Anspruch 1, dadurch gekennzeichnet, daß $R^1$ Chlor, $R^2$ Wasserstoff und $R^3$ Wasserstoff bedeuten.

3. Zubereitung gemäß Anspruch 1 oder 2, dadurch gekennzeichnet, daß der Angiontensin-Converting-Enzyme-Inhibitor die Formel III

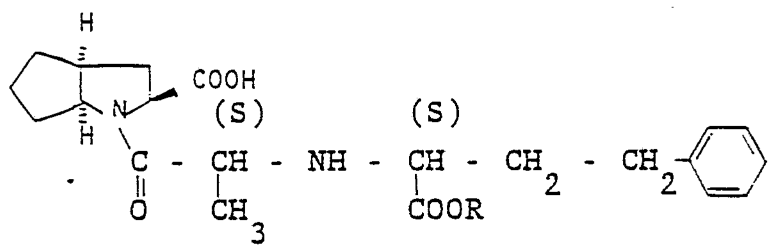

(III)

in welcher
R Wasserstoff, Methyl, Ethyl oder Benzyl bedeutet,
aufweist.

4. Zubereitung gemäß einem der Ansprüche 1 bis 3, dadurch gekennzeichnet, daß der Angiotensin-Converting-Enzyme-Inhibitor die Formel III, in welcher R Ethyl bedeutet, aufweist.

5. Zubereitung gemäß Anspruch 1 oder 2, dadurch gekennzeichnet, daß der Angiotensin-Converting-Enzyme-Inhibitor die Formel IV

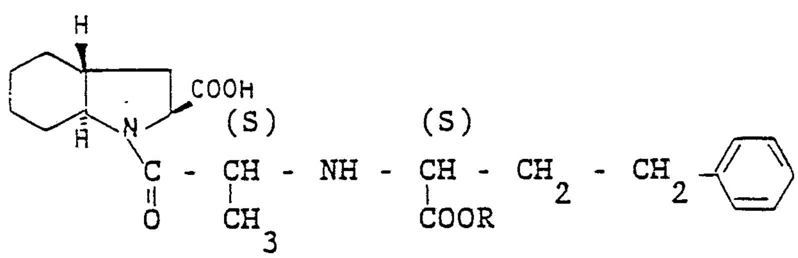

(IV)

in welcher
R Wasserstoff, Methyl, Ethyl oder Benzyl, vorzugsweise Ethyl bedeutet,
aufweist.

6. Zubereitung gemäß Anspruch 1 oder 2, dadurch gekennzeichnet, daß der Angiotensin-Converting-Enzyme-Inhibitor die Formel V

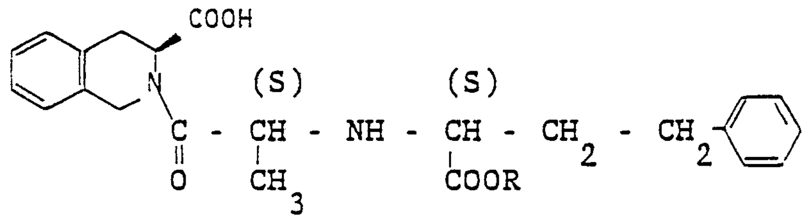

(V)

in welcher
R Wasserstoff, Methyl, Ethyl oder Benzyl, vorzugsweise Ethyl bedeutet,
aufweist.

7. Verfahren zur Herstellung einer Zubereitung gemäß einem der Ansprüche 1 bis 6, dadurch gekennzeichnet, daß man
a) einen Angiotensin-Converting-Enzyme-Inhibitor der Formel I oder dessen Salz und
b) ein Thiazid der Formel II oder dessen Salz
zusammen mit einem physiologisch annehmbaren Träger und gegebenenfalls weiteren Hilfs-oder Zusatzstoffen in eine geeignete Darreichungsform bringt.

8. Zubereitung gemäß einem der Ansprüche 1 bis 6 zur Anwendung als Heilmittel bei der Behandlung des Bluthochdrucks.

9. Erzeugnis, enthaltend
a) einen Angiotensin-Converting-Enzyme-Inhibitor der Formel I oder dessen physiologisch verträgliches Salz und
b) ein Thiazid der Formel II oder dessen physiologisch verträgliches Salz
als Kombinationspräparat zur gleichzeitigen, getrennten oder zeitlich abgestuften Anwendung bei der Behandlung des Bluthochdrucks.

10. Erzeugnis gemäß Anspruch 9, dadurch gekennzeichnet, daß seine Komponenten wie in einem der Ansprüche 2 bis 6 definiert sind.

11. Verwendung von Angiotensin-Converting-Enzyme-Inhibitoren der Formel I in Kombination mit Thiaziden der Formel II bei der Behandlung des Bluthochdrucks.

Patentansprüche für die folgenden Vertragstaaten : GR, AT, ES

1. Verfahren zur Herstellung einer pharmazeutischen Zubereitung, enthaltend
a) einen Angiotensin-Converting-Enzyme-Inhibitor der Formel I

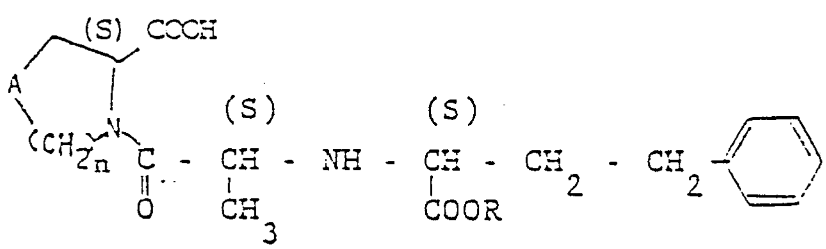

(I)

in welcher
A Cyclopentan-1,2-diyl, Cyclohexan-1,2-diyl oder o-Phenylen und
R Wasserstoff, Methyl, Ethyl oder Benzyl bedeuten und
n 0 oder 1 ist,
oder
dessen physiologisch verträgliches Salz und
b) ein Thiazid der Formel II



BAD ORIGINAL

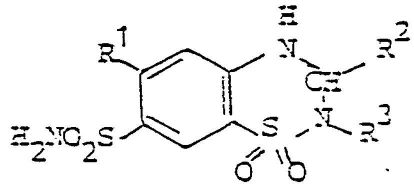

(II)

in welcher
$R^1$ Chlor oder Trifluormethyl,
$R^2$ Wasserstoff, Cyclopentylmethyl, Benzyl, Benzylthiomethyl, Isobutyl, Chlormethyl, Dichlormethyl oder 2,2,2-Trifluorethylthiomethyl und
$R^3$ Wasserstoff oder Methyl bedeuten,
oder dessen physiologisch verträgliches Salz, dadurch gekennzeichnet, daß man
a) einen Angiotensin-Converting-Enzyme-Inhibitor der Formel I oder dessen Salz und
b) ein Thiazid der Formel II oder dessen Salz
zusammen mit einem physiologisch annehmbaren Träger und gegebenenfalls weiteren Hilfs-oder Zusatzstoffen in eine geeignete Darreichungsform bringt.

2. Verfahren gemäß Anspruch 1, dadurch gekennzeichnet, daß man ein Thiazid der Formel II, worin $R^1$ Chlor, $R^2$ Wasserstoff und $R^3$ Wasserstoff bedeutet, verwendet.

3. Verfahren gemäß Anspruch 1 oder 2, dadurch gekennzeichnet, daß man einen Angiotensin-Converting-Enzyme-Inhibitor der Formel III

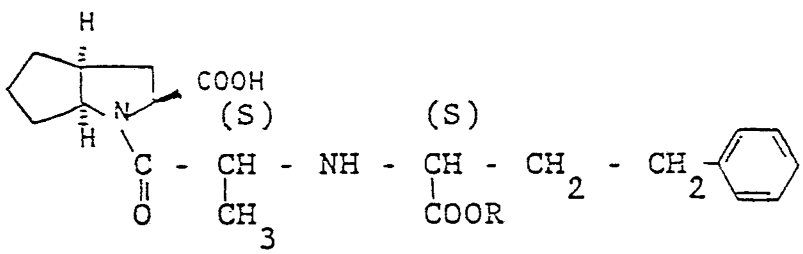

(III)

in welcher
R Wasserstoff, Methyl, Ethyl oder Benzyl bedeutet, verwendet.

4. Verfahren gemäß einem der Ansprüche 1 bis 3, dadurch gekennzeichnet, daß man einen Angiotensin-Converting-Enzyme-Inhibitoren der Formel III, in welcher R Ethyl bedeutet, verwendet.

5. Verfahren gemäß Anspruch 1 oder 2, dadurch gekennzeichnet, daß man einen Angiontensin-Converting-Enzyme-Inhibitoren der Formel IV

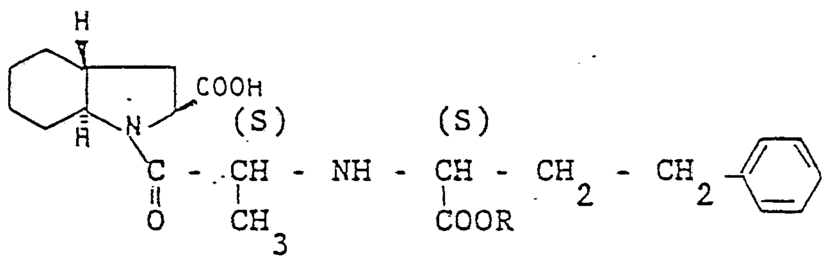

(IV)

in welcher
R Wasserstoff, Methyl, Ethyl oder Benzyl, vorzugsweise Ethyl bedeutet, verwendet.

6. Verfahren gemäß Anspruch 1 oder 2, dadurch gekennzeichnet, daß man einen Angiotensin-Converting-Enzyme-Inhibitoren der Formel V

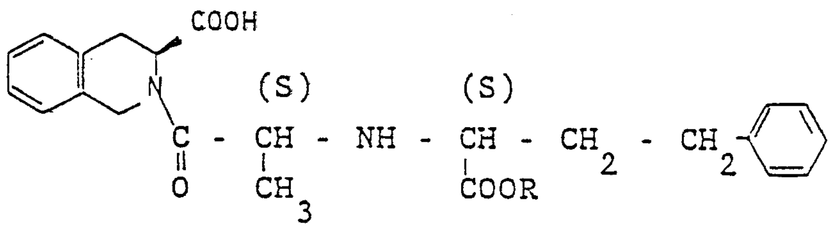

(V)

in welcher
R Wasserstoff, Methyl, Ethyl oder Benzyl, vorzugsweise Ethyl bedeutet, verwendet.

7. Verfahren zur Herstellung eines Erzeugnisses, enthaltend
a) einen Angiotensin-Converting-Enzyme-Inhibitor der Formel I oder dessen physiologisch verträgliches Salz und
b) ein Thiazid der Formel II oder dessen physioloigsch verträgliches Salz
als Kombinationspräparat zur gleichzeitigen, getrennten oder zeitlich abgestuften Anwendung bei der Behandlung des Bluthochdrucks; dadurch gekennzeichnet, daß man die beiden Komponenten jeweils zusammen mit einem physiologisch annehmbaren Träger und gegebenenfalls weiteren Hilfs-oder Zusatzstoffen in eine geeignete Darreichungsform bringt.

8. Verfahren gemäß Anspruch 7, dadurch gekennzeichnet, daß seine Komponenten wie in einem der Ansprüche 2 bis 6 definiert sind.

9. Verwendung von Angiotensin-Converting-Enzyme-Inhibitoren der Formel I in Kombination mit Thiaziden der Formel II bei der Behandlung des Bluthochdrucks



BAD ORIGINAL

Europäisches Patentamt

# EUROPÄISCHER RECHERCHENBERICHT

Nummer der Anmeldung

EP 87 11 3801

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | CHEMICAL ABSTRACTS, Band 100, Nr. 23, 4. Juni 1984, Seite 40, Zusammenfassung Nr. 185552f, Columbus, Ohio, US; P.S. CHAN et al.: "Acute antihypertensive synergism of angiotensin-converting enzyme inhibitors and diuretics", & FED. PROC., FED. AM. SOC. EXP. BIOL. 1984, 43(5), 1346-50<br>* Zusammenfassung *<br>--- | 1-11 | A 61 K 37/64 //<br>(A 61 K 37/64<br>A 61 K 31:54 ) |
| P,X | CHEMICAL ABSTRACTS, Band 107, Nr. 15, 12. Oktober 1987, Seite 37, Zusammenfassung Nr. 126749x, Columbus, Ohio, US; R.H.A. BECKER et al.: "Synergism of CE-inhibition and diuresis. A study with ramipril, piretanide and HCT in SHR", & CLIN. EXP. HYPERTENS., PART A 1987, A9(2-3), 357-60<br>* Zusammenfassung *<br>----- | 1-11 | |
| | | | **RECHERCHIERTE SACHGEBIETE (Int. Cl.4)** |
| | | | A 61 K |

Der vorliegende Recherchenbericht wurde für alle Patentansprüche erstellt

| Recherchenort | Abschlußdatum der Recherche | Prüfer |
|---|---|---|
| DEN HAAG | 06-01-1988 | BRINKMANN C. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)